# EUROPÄISCHE PATENTANMELDUNG

(11) **EP 1 746 163 A1**
(43) Veröffentlichungstag der Anmeldung: **24.01.2007**
(21) Anmeldenummer: 05090220.4
(22) Anmeldetag: 22.07.2005
(51) Int. Cl.: C12N 15/82, C12N 15/54, A01H 5/00, A01H 5/10

(54) **Überexpression von löslicher Stärkesynthase II in Pflanzen**

(71) Anmelder: Bayer CropScience GmbH, 65929 Frankfurt/Main (DE)
(72) Erfinder: Frohberg, Claus, 14532 Kleinmachnow (DE)
(74) Vertreter: Kossmann, Jochen

(57) **Zusammenfassung**

Die vorliegende Erfindung betrifft ein Verfahren zur Erhöhung des Phosphatgehalts von Stärken genetisch modifizierter Pflanzenzellen im Vergleich zu Stärken von entsprechenden Wildtyppflanzenzellen durch Einführung eines fremden Nukleinsäuremoleküls, welches für eine lösliche Stärkesynthase II kodiert. Ferner betrifft die vorliegende Erfindung die Überexpression dieser löslichen Stärkesynthase II in den genetisch modifizierten Pflanzenzellen.

## Beschreibung

Die vorliegende Erfindung betrifft ein Verfahren zur Erhöhung des Phosphatgehalts von Stärken genetisch modifizierter Pflanzenzellen im Vergleich zu Stärken aus entsprechenden genetisch nicht modifizierten Wildtyppflanzenzellen, das dadurch gekennzeichnet ist, dass eine Pflanzenzelle genetisch modifiziert wird durch die Einführung eines fremden Nukleinsäuremoleküls, welches eine lösliche Stärkesynthase II kodiert, und diese Stärkesynthase II überexprimiert wird.

Neben Ölen, Fetten und Proteinen stellen Polysaccharide die wesentlichen nachwachsenden Rohstoffe aus Pflanzen dar. Eine zentrale Stellung bei den Polysacchariden nimmt neben Cellulose die Stärke ein, die einer der wichtigsten Speicherstoffe in höheren Pflanzen ist.

Das Polysaccharid Stärke ist ein Polymer aus chemisch einheitlichen Grundbausteinen, den Glucosemolekülen. Es handelt sich dabei jedoch um ein sehr komplexes Gemisch aus unterschiedlichen Molekülformen, die sich hinsichtlich ihres Polymerisationsgrades des Auftretens von Verzweigungen der Glucoseketten und deren Kettenlängen unterscheiden, die darüber hinaus modifiziert, z.B. phosphoryliert sein können. Daher stellt Stärke keinen einheitlichen Rohstoff dar. Man unterscheidet insbesondere die Amylose, ein im wesentlichen unverzweigtes Polymer aus α-1,4-glycosidisch verknüpften Glucosemolekülen, vom Amylopektin, die ihrerseits ein komplexes Gemisch aus unterschiedlich verzweigten Glucoseketten darstellt. Die Verzweigungen kommen dabei durch das Auftreten von zusätzlichen α-1,6-glycosidischen Verknüpfungen zustande. In typischen, für die industrielle Stärkeproduktion verwendeten Pflanzen, wie z.B. Mais, Weizen oder Kartoffel, besteht die synthetisierte Stärke zu ca. 20% - 25% aus Amylose-Stärke und zu ca. 75% - 80% aus Amylopektin-Stärke.

Die funktionellen Eigenschaften der Stärke werden neben dem Amylose/Amylopektin-Verhältnis und dem Phosphatgehalt stark beeinflusst durch das Molekulargewicht, das Muster der Seitenkettenverteilung, den Gehalt an Ionen, den Lipid- und Proteingehalt, die mittlere Stärkekorngröße sowie die Stärkekorn-Morphologie etc. Als wichtige, funktionelle Eigenschaften sind hierbei beispielsweise zu nennen: die Löslichkeit, das Retrogradationsverhalten, das Wasserbindevermögen, die Filmbildungseigenschaften, die Viskosität, die Verkleisterungseigenschaften, die Gefrier-/Tau-Stabilität, die Säurestabilität, die Gelfestigkeit etc.

Derzeit ist es nicht möglich, den Gehalt an kovalent gebundenem Stärkephosphat in Pflanzen allein durch züchterische Maßnahmen zu beeinflussen.

Eine Alternative zu züchterischen Verfahren besteht in der gezielten Modifikation stärkeproduzierender Pflanzen durch gentechnologische Methoden. Voraussetzung hierfür ist jedoch die Identifizierung und Charakterisierung der an der Stärkesynthese und/oder Stärkemodifikation beteiligten Enzyme sowie die Isolierung der diese Enzyme kodierenden Nukleinsäuremoleküle und deren anschließende funktionelle Analyse in transgenen Pflanzen.

Die biochemischen Synthesewege, die zum Aufbau von Stärke führen, sind lediglich im groben bekannt. Die genauen Mechanismen, die zum Aufbau der Stärkekörner und zur Synthese der Stärke führen, sind bislang jedoch nicht abschließend geklärt und somit weiterhin Gegenstand der Forschung.

Die Stärkesynthese in pflanzlichen Zellen findet in den Plastiden statt. In photosynthetisch aktiven Geweben sind dies die Chloroplasten, in photosynthetisch inaktiven, stärkespeichernden Geweben die Amyloplasten. Wichtige an der Stärkesynthese beteiligte Enzyme sind die R1-Proteine (=alpha-Glucan-Wasser-Dikinase, E.C. 2.7.9.4; Lorberth et al. (1998) Nature Biotechnology 16: 473-477), die Stärkesynthasen sowie die Verzweigungsenzyme (= "Branching enzymes" = BE; siehe, z.B. Kossmann et al., 1991, Mol. Gen. Genet. 230, 39-44; Safford et al., 1998, Carbohydrate Polymers 35, 155-168; Jobling et al. 1999, The Plant Journal 18(2): 163-171). Verzweigungsenzyme katalysieren die Einführung von α-1,6-Verzweigungen in lineare α-1,4-Glucane.
Bei den Stärkesynthasen sind verschiedene Klassen beschrieben, die alle eine Polymerisierungsreaktion durch Übertragung eines Glucosylrestes von ADP-Glucose auf α-1,4-Glucane katalysieren. Stärkesynthasen (EC2.4.1.21) können in zwei Klassen eingeteilt werden: die Stärkekorn-gebundenen Stärkesynthasen ("granule-bound starch synthases I"; GBSS I) und die löslichen Stärkesynthasen ("soluble starch synthases";' SSS, auch als "SS" bezeichnet). Diese Unterscheidung ist nicht in jedem Fall eindeutig zu treffen, da einige der Stärkesynthasen sowohl stärkekorngebunden als auch in löslicher Form vorliegen (Denyer et al., Plant J. 4 (1993), 191-198; Mu et al., Plant J. 6 (1994), 151-159).

Im Gegensatz zur GBSSI, die zur Synthese von Amylose führt, ist über die exakte enzymatische Funktion der verschiedenen Klassen der löslichen Stärkesynthase an der Stärkebiosynthese noch wenig bekannt.

Die biochemische Charakterisierung führte zu einer Identifizierung von Proteinen der löslichen Stärkesynthase mit Molekulargewichten zwischen ca. 60 bis ca. 180 kDa. Die Klonierung von cDNAs, die für Stärkesynthasen codieren, ermöglichte eine Einteilung in verschiedene Klassen, die aufgrund von Sequenzhomologien sowie aufgrund der funktionellen Eigenschaften der (löslichen) Stärkesynthasen festgelegt wurden.

In höheren Pflanzen wurden bislang acht Klassen von Stärkesynthasen identifiziert (u.a. nach Li et al. (2003) Funct. Intergr. Genomics 3:76-85):
- Stärkekorngebundene Stärkesynthase I (Granule-Bound Starch Synthase I = GBSS I) (Reis: z.B. Okagaki (1992) Plant Mol Biol. 19:513-516; Kartoffel: van der Leij et al. (1991) Mol. Gen Genet. 228:240-248; Mais: z.B. Kloesgen et al. (1986) Mol. Gen Genet. 203:237-244);
- lösliche Stärkesynthase I (= SSI; Reis: Baba et al. (1993) Plant Physiol. 103:565-573; Kartoffel: Kossmann et al. (1999) Planta 208: 503-511; Mais: Knight et al. (1998) Plant J.' 14:613-622);
- lösliche Stärkesynthase II (= SSII; Erbse: Dry et al. (1992) Plant J. 2:193-202, Kartoffel: Edwards et al. (1995) Plant J 8: 283-294, Mais: Harn et al, (1998) Plant Mol. Biol. 37(4): 639-649; Weizen: Walter et al. (1996) Genbank Acc. U66377; Reis: Yamamoto und Sasaki (1997) Plant Mol. Biol. 35:135-144 und Gerste: Li et al. (2003), Funct. Integr. Genomics 3: 76-85);
- lösliche Stärkesynthase III (= SSIII; Kartoffel: Abel et al. (1996) Plant J 10:981-991; Futtererbse: GenBank Acc.No AJ225088);
- lösliche Stärkesynthase IV (= SSIV; Weizen: GenBank Acc.No AY044844);
- lösliche Stärkesynthase V (= SSV; Futtererbse: GenBank Acc.No VUN006752; Arabidopsis: GenBank Acc.No AL021713; Mais: WO 97/26362) und
- dull (Gao et al. (1998) Plant Cell 10:399-412 ;
- lösliche Stärkessynthase VI (= SSVI; Mais: WO 01/12826).

Der Gehalt an kovalent gebundenem Stärkephosphat variiert je nach Pflanzenart. So synthetisieren z.B. bestimmte Maismutanten eine Stärke mit erhöhtem Gehalt an Stärkephosphat (waxy-Mais 0,002% und Hoch-Amylose-Mais 0,013%), während herkömmliche Maissorten nur Spuren von Stärkephosphat aufweisen. Ebenfalls geringe Mengen an Stärkephosphat findet man in Weizen (0,001%), während in Hafer und Hirse' kein Stärkephosphat nachgewiesen werden konnte. In Reis-Mutanten wurde ebenfalls weniger Stärkephosphat gefunden (waxy-Reis 0,003%) als in herkömmlichen Reissorten (0,013%). Signifikante Mengen von Stärkephosphat wurden in Knollen- oder Wurzelspeichestärke synthetisierenden Pflanzen wie z.B. Tapioca (0,008%), Süßkartoffel (0,011%), Pfeilwurz (0,021%) oder Kartoffel (0,089%) nachgewiesen. Diese prozentualen Werte für den Stärkephosphatgehalt beziehen sich jeweils auf das Trockengewicht der Stärke und sind von Jane et al. (1996, Cereal Foods World 41 (11), 827-832) ermittelt worden. Untersuchungen an SSI-antisense Kartoffeln ergaben eine Erhöhung des Phosphatgehaltes um 30-70% gegenüber dem Wildtyp (WO 96/15248).
In WO 00/08184 sind Pflanzen beschrieben, bei welchen sowohl die Aktivitäten der Stärkesynthase III (=SSIII) als auch des Branching enzymes I (= BEI) reduziert ist. Stärke aus solchen Pflanzen weist im Vergleich zu Stärke aus Wildtyp-Pflanzen einen erhöhten Phosphatgehalt auf. Weizenpflanzen, welche durch Überexpression eines R1 Gens aus Kartoffel eine erhöhte Aktivität eines R1 Proteins und erhöhten Phosphatgehalt der Stärke aufweisen, sind in der internationalen Patentanmeldung WO 02/34923 beschrieben.

Die Verteilung des Phosphates in von Pflanzen synthetisierter (nativer) Stärke zeichnet sich im Allgemeinen dadurch aus, dass etwa 30% bis 40% der Phosphatreste in C-3-Position und etwa 60% bis 70% der Phosphatreste in C-6-Position der Glucosemoleküle kovalent gebunden sind (Blennow et al., 2000, Int. J. of Biological Macromolecules 27: 211-218). Im Unterschied dazu weisen chemisch phosphorylierte Stärken zusätzlich Phosphatreste in C-2-Position der Glucosemoleküle kovalent gebunden auf, da die chemische Reaktion ungerichtet verläuft.

Eine Übersicht zu nativen Stärken, isoliert aus verschiedenen Pflanzenspezies, die eine Veränderung von an der Stärkebiosynthese beteiligten Enzymen aufweisen, findet sich bei Kossmann und Lloyd (2000, Critical Reviews in Plant Sciences 19(3), 171- 226).

Bislang war nicht bekannt, wie sich die Erhöhung der Genexpression der löslichen Stärkesynthase II auf die Stärkeeigenschaften in Pflanzen auswirkt. Ein hoher Phosphatgehalt ist erwünscht, da er zu veränderten physiko-chemischen Eigenschaften der Stärke und damit zu neuen Anwendungsmöglichkeiten der Stärke führt.

Aufgabe der vorliegenden Erfindung ist es daher, ein Verfahren bereitzustellen, mit dem der Phosphatgehalt von Stärken von Pflanzen in vivo erhöht werden kann.

Diese Aufgabe wird durch die Bereitstellung der in den Patentansprüchen bezeichneten Ausführungsformen gelöst.

Die vorliegende Erfindung betrifft ein Verfahren zur Erhöhung des Phosphatgehaltes von Stärken genetisch modifizierter Pflanzenzellen auf 150 bis 500% im Vergleich zu Stärken aus entsprechenden genetisch nicht modifizierten Wildtyppflanzenzellen (100%), dadurch gekennzeichnet, dass
a) eine Pflanzenzelle genetisch modifiziert wird durch die Einführung eines fremden Nukleinsäuremoleküls kodierend eine lösliche Stärkesynthase II und
b) diese lösliche Stärkesynthase II überexprimiert wird.

Unter dem Begriff "Phosphatgehalt von Stärke" sollen im Zusammenhang mit der vorliegenden Erfindung kovalent an die Glucosemonomere der Stärke gebundene Phosphatgruppen verstanden werden.

Unter dem Begriff "Erhöhung des Phosphatgehaltes von Stärken" versteht man im Zusammenhang mit der vorliegenden Erfindung eine Erhöhung des Phosphatgehaltes in C-6-Position auf 150-500%, bevorzugt auf 160-400% und besonders bevorzugt auf 170-380 % im Vergleich zu Stärke aus entsprechenden Wildtyppflanzenzellen (100%).

Unter dem Begriff "Phosphatgehalt in C6-Position" ist im Zusammenhang mit der vorliegenden Erfindung der Gehalt an Phosphatgruppen zu verstehen, die an Kohlenstoffatomposition "6" der Glukosemonomere der Stärke gebunden sind. Grundsätzlich können in der Stärke in vivo die Positionen C3 und C6 der Glukoseeinheiten phosphoryliert sein. Die Bestimmung des Phosphatgehaltes in C6-Position (= C-6-P-Gehalt) erfolgt im Zusammenhang mit der vorliegenden Erfindung über eine Glukose-6-phosphat-Bestimmung mittels des weiter unten beschriebenen optisch-enzymatischen Tests (nach Nielsen et al., 1994, Plant Physiol. 105, 111-117).

Äußerst überraschend war bei der vorliegenden Erfindung, dass eine Erhöhung des Phosphatgehaltes auf deutlich mehr als 150 % im Vergleich zum Wildtyp (100%) möglich war.

Die Erhöhung des Phosphatgehaltes von Stärken erfolgt im Zusammenhang mit der vorliegenden Erfindung in vivo, nicht in vitro, wie beispielsweise durch chemische Phosphorylierung bereits extrahierter Stärke. Daher hat die vorliegende Erfindung den Vorteil, dass chemische Agenzien zur chemischen Phosphorylierung eingespart werden können.

Im Zusammenhang mit der vorliegenden Erfindung bedeutet der Begriff "genetisch modifizierte Pflanzenzelle", dass die Pflanzenzelle genetisch modifiziert ist, wobei die genetische Modifikation zur Erhöhung der Aktivität einer löslichen Stärkesynthase II (=SSII) führt im Vergleich zur SSII-Aktivität einer entsprechenden genetisch nicht modifizierten Wildtyppflanzenzelle.

Der Begriff "Wildtyppflanzenzelle" bedeutet im Zusammenhang mit der vorliegenden Erfindung, dass es sich um Pflanzenzellen handelt, die als Ausgangsmaterial für das erfindungsgemäße Verfahren dienen, d.h. deren genetische Information, abgesehen von der eingeführten genetischen Modifikation, die zu einer Erhöhung der Aktivität einer' löslichen Stärkesynthase II (=SSII) führt, derjenigen einer genetisch modifizierten Pflanzenzelle entspricht.

Der Begriff "entsprechend" bedeutet im Zusammenhang mit der vorliegenden Erfindung, dass beim Vergleich von mehreren Gegenständen die betreffenden Gegenstände, die miteinander verglichen werden, unter gleichen Bedingungen gehalten wurden. Im Zusammenhang mit der vorliegenden Erfindung bedeutet der Begriff "entsprechend" mit Bezug auf Wildtyppflanzenzellen, dass die Pflanzenzellen, die miteinander verglichen werden, unter gleichen Kulturbedingungen aufgezogen wurden und dass sie vorzugsweise das gleiche (Kultur-) Alter aufweisen.

Unter dem Begriff "Kulturalter" ist die Zeitdauer zu verstehen, die ein (pflanzlicher) Organismus in einem Nährmedium verbringt/wächst. Dies kann z.B. der Zeitraum von Aussaat bis Ernte als auch der Zeitraum der Kultivierung pflanzlicher Zellen in einem Gewebekulturmedium bis zu einem bestimmten Entwicklungsstadium sein. Im Zusammenhang mit der vorliegenden Erfindung ist damit gemeint, dass die Pflanzenzellen, die miteinander verglichen werden, denselben Entwicklungszeitraum' unter den gleichen Kulturbedingungen verbringen.

Unter dem Begriff "fremdes Nukleinsäuremolekül" ist im Zusammenhang mit der vorliegenden Erfindung ein solches Molekül zu verstehen, das entweder natürlicherweise in entsprechenden Wildtyppflanzenzellen nicht vorkommt oder das in der konkreten räumlichen Anordnung nicht natürlicherweise in entsprechenden Wildtyppflanzenzellen vorkommt oder das an einem Ort im Genom der Pflanzenzelle lokalisiert ist, an dem es natürlicherweise nicht vorkommt. Bevorzugt ist das fremde Nukleinsäuremolekül/Polynukleotid ein rekombinantes Molekül, das aus verschiedenen Elementen besteht, deren Kombination oder spezifische räumliche Anordnung natürlicherweise in pflanzlichen Zellen nicht auftritt. Dies lässt sich beispielsweise mit Hilfe einer Southern Blot-Analyse nachprüfen.

Im Zusammenhang mit dem erfindungsgemäßen Verfahren umfasst die genetische Modifikation vorzugsweise das Einführen mindestens eines fremden Nukleinsäuremoleküls, kodierend eine lösliche Stärkesynthase II (SSII), in das Genom einer Pflanzenzelle, wobei besagtes Einführen mindestens eines fremden Nukleinsäuremoleküls dazu führt, dass die Stärke, erhältlich aus der genetisch modifizierten Pflanzenzelle und der daraus regenerierten Pflanze, aufgrund der Expression der eingeführten SSII einen erhöhten Gehalt an Phosphat in C6-Position aufweist im Vergleich zu entsprechenden genetisch nicht modifizierten Wildtyppflanzenzellen.

Für die Einführung von DNA in eine pflanzliche Wirtszelle stehen eine Vielzahl von Techniken zur Verfügung. Diese Techniken umfassen die Transformation pflanzlicher Zellen mit T-DNA unter Verwendung von *Agrobacterium tumefaciens* oder *Agrobacterium rhizogenes* als Transformationsmittel, die Fusion von Protoplasten, die Injektion, die Elektroporation von DNA, die Einbringung der DNA mittels des biolistischen Ansatzes sowie weitere Möglichkeiten.

Die Transformation monokotyledoner Pflanzen mittels auf Agrobakterium-Transformation basierender Vektoren wurde beispielsweise beschrieben in Chan et al., 1993, Plant Mol. Biol. 22, 491-506; Hiei et al., 1994, Plant J. 6, 271-282; Deng et al., 1990, Science in China 33, 28-34; Wilmink et al., 1992, Plant Cell Reports 11, 76-80; May et al., 1995, Bio/Technology 13, 486-492; Conner und Domisse, 1992, Int. J. Plant Sci. 153, 550-555 und in Ritchie et al, 1993, Transgenic Res. 2, 252-265. Alternative Systeme zur Transformation monokotyledoner Pflanzen sind: die Transformation mittels des biolistischen Ansatzes (Wan und Lemaux, 1994, Plant Physiol. 104, 37-48; Vasil et al., 1993, Bio/Technology 11, 1553-1558; Ritala et al., 1994, Plant Mol. Biol. 24, 317-325; Spencer et al., 1990, Theor. Appl. Genet. 79, 625-631), die Protoplasten-Transformation, die Elektroporation von partiell permeabilisierten Zellen sowie die Einbringung von DNA mittels Glasfasern. Die erfolgreiche Transformation verschiedener Getreidearten wurde beschrieben z.B. für Gerste (Wan und Lemaux, supra; Ritala et al., supra; Krens et al., 1982, Nature 296, 72-74), für Weizen (Nehra et al., 1994, Plant J. 5, 285-297), Reis (Ishida et al., 1996, Nature Biotechnology 14 (6): 745-750) sowie Mais (Koziel et al. (1993), Biotechnology 11: 194-200).

Die Regeneration der genetisch modifizierten Pflanzenzellen des erfindungsgemäßen Verfahrens führt zur Herstellung genetisch modifizierter Pflanzen, deren genetische Information der einer entsprechenden genetisch nicht modifizierten Wildtyppflanze entspricht und die die gleiche eingeführte genetische Modifikation zur Erhöhung der Aktivität einer löslichen Stärkesynthase II enthält, die bereits die genetisch modifizierten Pflanzenzellen des erfindungsgemäßen Verfahrens aufweisen.

Die genetisch modifizierten Pflanzen, deren Stärke durch das erfindungsgemäße Verfahren modifiziert wird, können zu jeder beliebigen Pflanzenspezies gehören, d.h. sowohl zu den monokotyledonen als auch zu dikotyledonen Pflanzen. Bevorzugt handelt es sich um landwirtschaftliche Nutzpflanzen, d.h. Pflanzen, die vom Menschen kultiviert werden für Zwecke der Ernährung oder für technische, insbesondere industrielle Zwecke und deren Zellen. Vorzugsweise wird das erfindungsgemäße Verfahren in stärkespeichernden Pflanzen wie z.B. Erbsen, Kartoffel, Süßkartoffel, Maniok, Mais und Reis angewendet.

In einer besonders bevorzugten Ausführungsform wird das erfindungsgemäße Verfahren mit Reispflanzen durchgeführt.

Unter dem Begriff "lösliche Stärkesynthase II" (= SSII-Protein) ist im Zusammenhang mit der vorliegenden Erfindung die Klasse II von löslichen Stärkesynthase-Proteinen (ADP-Glukose 1,4-α-D-glukan 4-α-D-glucosyltransferase; EC 2.4.1.21) zu verstehen. Lösliche Stärkesynthasen katalysieren eine Glycosylierungsreaktion, bei der Glukosereste des Substrates ADP-Glukose unter Bildung einer α-1,4-Verknüpfung auf α-1,4-verknüpfte Glukanketten übertragen werden (ADP-Glukose + {(1,4)- α-D-glucosyl}(N) <=> ADP + {(1,4)- α-D-glucosyl}(N+1)).

SSII-Proteine weisen in ihrem Aufbau eine Abfolge bestimmter Domänen auf. SSII-Proteine haben am "N"-Terminus ein Signalpeptid für den Transport in Plastiden. In Richtung C-Terminus folgen eine N-terminale Region und eine katalytische Domäne. (Li et al., 2000, Plant Physiology 123, 613-624). Weitere Analysen, basierend auf Primärsequenzvergleichen (http://hits.isb-sib.ch/cgi-bin/PFSCAN) verschiedener SSII-Proteine ergaben, dass SSII Proteine drei spezifische Domänen aufweisen. Diese Domänen umfassen die Aminosäuren, die von den Nukleotiden codiert werden der bp 1190 bis 1279 (= Region 1), den bp 1493 bis 1612 (Region 2) und den bp 2147 bis 2350 (Region 3) der in Seq ID Nr. 1 dargestellten Sequenz des SSII-Gens aus Weizen.

Unter einem SSII-Protein soll daher im Zusammenhang mit der vorliegenden Erfindung eine lösliche Stärkesynthase verstanden werden, deren Aminosäuresequenz mit der unter Seq ID No. 3 angegebenen Region 1 eine Identität von mindestens 86%, bevorzugt mindestens 93% und besonders bevorzugt 100% und mit der unter Seq. ID No. 4 angegebenen Region 2 eine Identität von mindestens 83%, bevorzugt mindestens 86% und besonders bevorzugt 100% und mit der unter Seq. ID No. 5 angegebenen Region 3 eine Identität von mindestens 70%, vorzugsweise mindestens 82%, bevorzugt 86%, besonders bevorzugt 98% und insbesondere bevorzugt von 100% aufweist.

Unter dem Begriff "Identität" soll im Zusammenhang mit der vorliegenden Erfindung die Anzahl der übereinstimmenden Aminosäuren (Identität) mit Aminosäuren anderer Proteine verstanden werden, ausgedrückt in Prozent. Bevorzugt wird die Identität mit Hilfe von Computerprogrammen ermittelt. Weisen Sequenzen, die miteinander verglichen werden, unterschiedliche Längen auf, ist die Identität so zu ermitteln, dass die Anzahl an Aminosäuren, welche die kürzere Sequenz mit der längeren Sequenz gemeinsam hat, den prozentualen Anteil der Identität bestimmt. Vorzugsweise wird die Identität mittels des bekannten und der Öffentlichkeit zur Verfügung stehenden Computerprogramms ClustalW (Thompson et al., (1994) Nucleic Acids Research 22: 4673-4680) ermittelt.

ClustalW wird öffentlich zur Verfügung gestellt von Julie Thompson (Thompson@EMBL-Heidelberg.DE) und Toby Gibson (Gibsono@EMBL-Heidelberg.DE), European Molecular Biology Laboratory, Meyerhofstrasse 1, D - 69117 Heidelberg. ClustalW kann ebenfalls von verschiedenen Internetseiten, u.a. beim IGBMC (Institut de Génétique et de Biologie Moléculaire et Cellulaire, B.P.163, 67404 Illkirch Cedex, France; ftp://ftp-igbmc.u-strasbg.fr/pub/) und beim EBI (ftp://ftp.ebi.ac.uk/pub/software/), sowie bei allen gespiegelten Internetseiten des EBI (European Bioinformatics Institute, Wellcome Trust Genome Campus, Hinxton, Cambridge CB10 1SD, UK), heruntergeladen werden.

Vorzugsweise wird das ClustalW Computerprogramm der Version 1.8 benutzt, um die Identität zwischen hierin beschriebenen Proteinen und anderen Proteinen zu bestimmen. Dabei sind folgende Parameter einzustellen: KTUPLE=1, TOPDIAG=5, WINDOW=5, PAIRGAP=3, GAPOPEN=10, GAPEXTEND=0.05, GAPDIST=8, MAXDIV=40, MATRIX=GONNET, ENDGAPS(OFF), NOPGAP, NOHGAP.

Vorzugsweise wird das ClustalW Computerprogramm der Version 1.8 benutzt, um die Identität zwischen den Nukleotidsequenzen der hierin beschriebenen Nukleinsäuremoleküle und der Nukleotidsequenz von anderen Nukleinsäuremolekülen zu bestimmen. Dabei sind folgende Parameter einzustellen: KTUPLE=2, TOPDIAGS=4, PAIRGAP=5, DNAMATRIX:IUB, GAPOPEN=10, GAPEXT=5, MAXDIV=40, TRANSITIONS: unweighted.

In einer bevorzugten Ausführungsform ist das erfindungsgemäße Verfahren weiterhin dadurch gekennzeichnet, dass es sich bei dem fremden Nukleinsäuremolekül um die kodierende Region einer heterologen löslichen Stärkesynthase II handelt.

Unter einer "heterologen löslichen Stärkesynthase II" ist im Zusammenhang mit der vorliegenden Erfindung eine lösliche Stärkesynthase II zu verstehen, die natürlicherweise nicht in der Pflanzenzelle vorkommt, sondern deren codierende DNA-Sequenz mittels gentechnischer Methoden, wie z.B. Transformation der Zelle, in die Zelle eingeführt wird. Dabei stammt die codierende DNA-Sequenz aus einer anderen Pflanzenart als die transformierte Pflanzenzelle bzw. Pflanze oder steht nicht unter der Kontrolle ihres eigenen Promotors. Vorzugsweise stammt die codierende DNA-Sequenz aus einer anderen Pflanzengattung als die transformierte Pflanzenzelle bzw. Pflanze.

Unter dem Begriff "Pflanzengattung" ist im Zusammenhang mit der vorliegenden Erfindung eine hierarchische Stufe der biologischen Systematik zu verstehen. Eine Gattung enthält eine oder mehrere Arten. Ein Beispiel einer Gattung ist *Triticum* L*.* (Weizen). Alle Arten innerhalb einer Gattung haben immer einen zweiteiligen (binominalen) Namen, der neben der Gattungsbezeichnung noch ein Art-Epipheton enthält. *Triticum aestivum* L. (der Weichweizen) ist danach eine Art der Gattung *Triticum.*

In einer weiteren bevorzugten Ausführungsform der Erfindung steht das eingeführte fremde Nucleinsäuremolekül kodierend eine lösliche Stärkesynthase II unter der Kontrolle eines endospermspezifischen Promotors. Mit Hilfe endospermspezifischer Promotoren ist es möglich, die Transkriptmenge der fremden Nukleinsäuremoleküle kodierend eine lösliche Stärkesynthase II im Endosperm im Vergleich zum Endosperm entsprechender Wildtyppflanzen zu erhöhen.

Bevorzugt werden Promotoren für eine endosperm-spezifische Expression verwendet, wie z. B. der Glutelinpromotor (Leisy et al. (1990) Plant Mol. Biol. 14: 41-50; Zheng et al. (1993) Plant J. 4:357-366), der HMG-Promotor aus Weizen (Anderson et al. (1989) Nucleic Acid Res 17:461-462), der USP-Promotor (Bäumlein et al. (1991) Mol. Gen Genetics 225: 121-128), der Phaseolinpromotor (Kawagoe und Murai (1992) Plant J 2 (6):927-36), Promotoren von Zein-Genen aus Mais (Pedersen et al., (1982) Cell 29: 1015-1026; Quatroccio et al., (1990) Plant Mol. Biol. 15 : 81-93) oder der shrunken-1-Promotor (sh-1) aus Mais (Werr et al. (1985) EMBO J. 4:1373-1380) verwendet.
Besonders bevorzugt wird der Globulin-Promotor aus Reis (Nakase et al. (1996) Gene 170(2):223-226) verwendet.

Unter dem Begriff "SSII-Gen" ist im Zusammenhang mit der vorliegenden Erfindung ein Nukleinsäuremolekül oder Polynukleotid (DNA, cDNA) zu verstehen, welches für eine "lösliche Stärkesynthase II" codiert. In einer bevorzugten Ausführungsform stammt das SSII-Gen aus einer monokotyledonen Pflanze.

Das erfindungsgemäße Verfahren ist in einer weiteren bevorzugten Ausführungsform dadurch gekennzeichnet, dass eine lösliche Stärkesynthase II aus einer Monokotylenpflanze verwendet wird. In einer besonders bevorzugten Ausführungsform wird die SSII durch die codierende Region der unter Seq ID No. 1 dargestellten Nukleotidsequenz codiert oder weist die unter Seq. ID No. 2 dargestellte Aminosäuresequenz auf.

Der Begriff "überexprimiert" bedeutet im Rahmen der vorliegenden Erfindung eine Erhöhung der enzymatischen Aktivität von SSII-Proteinen in den genetisch modifizierten Pflanzenzellen im Vergleich zu entsprechenden genetisch nicht modifizierten WildtypPflanzenzellen. Ferner bedeutet der Begriff "überexprimiert" im Rahmen der vorliegenden Erfindung auch, dass Pflanzen oder Pflanzenzellen, die natürlicherweise keine nachweisbare SSII-Aktivität aufweisen, nach erfindungsgemäßer genetischer Modifikation, bei der ein fremdes Nucleinsäuremolekül codierend eine lösliche Stärkesynthase II in das Genom einer Pflanzenzelle eingeführt wird, eine im Zymogramm nachweisbare SSII-Aktivität aufweisen. Die Erhöhung der enzymatischen Aktivität von SSII-Proteinen in den Zellen wird vorzugsweise mit Hilfe von Zymogrammen, wie weiter unten beschrieben ("Bestimmung der SSII-Aktivität mittels Aktivitätsgel") bestimmt.

Eine Erhöhung der SSII Aktivität bedeutet dabei eine Erhöhung der SSII-Aktivität im Vergleich zu entsprechenden nicht genetisch modifizierten Zellen auf mindestens 200%, insbesondere auf 350-2000%, bevorzugt auf 600-1500% und besonders bevorzugt auf 700-1200%. Eine "Erhöhung der SSII-Aktivität" bedeutet im Zusammenhang mit der vorliegenden Erfindung auch, dass Pflanzen oder Pflanzenzellen, die keine nachweisbare SSII-Aktivität aufweisen, nach erfindungsgemäßer genetischer Modifikation, bei der ein fremdes Nucleinsäuremolekül codierend eine lösliche Stärkesynthase in das Genom einer Pflanzenzelle eingeführt wird, eine nachweisbare SSII-Aktivität aufweisen.

### Material und Methoden

In den Beispielen wurden die folgenden Methoden verwendet. Diese Methoden können zur Durchführung der erfindungsgemäßen Verfahren verwendet werden, sie stellen konkrete Ausführungsformen der vorliegenden Erfindung dar, beschränken die vorliegende Erfindung jedoch nicht auf diese Methoden. Dem Fachmann ist bekannt, dass er durch Modifikation der beschriebenen Methoden und/oder durch Ersetzen einzelner Methodenteile durch alternative Methodenteile die Erfindung in gleicher Weise ausführen kann. Eine Ausnahme bildet lediglich die Methode der "Bestimmung des Gehaltes an Stärke-gebundenem Glucose-6-Phosphat", die im Zusammenhang mit der vorliegenden Erfindung immer in der unter 7) beschriebenen Weise durchzuführen ist.
**1) Pflanzenmaterial**
   Reispflanzen:
   *Oryza sativa* var. japonica, Linien M202
**2) Herkunft der zur Transformation verwendeten Sequenzen und Konstrukte**
   Zur Transformation von Reis wurde die Sequenz Ta_SS2 aus Weizen verwendet. Isolierung und Klonierung erfolgte wie in WO 97-45545 beschrieben.
   Der verwendete Transformationsvektor AH32-191 ist in Beispiel 1 beschrieben.
**3) Transformation und Regeneration von Reispflanzen**
   Reispflanzen wurden nach der von Ishida et al. (1996, Nature BioTechnology, 14 (6): 745-750) beschriebenen Methode transformiert.
**4) Prozessierung von Reiskörnern**
   Zur Erzeugung von ausreichenden Mengen an Untersuchungsmaterial wurden Reispflanzen unter Gewächshausbedingungen angezogen und nach Erreichen vollständiger Reife geerntet. Zum weiteren Trocknen wurden die reifen Reiskörner für 3-7 Tage bei 37°C gelagert.
   Nachfolgend wurden die Körner mittels eines Entspelzers (Laboratory Paddy sheller, Grainman, Miami, Florida, USA) von den Spelzen befreit und der erhaltene braune Reis wurde durch 1-minütiges Polieren (Pearlest Rice Polisher, Kett, Villa Park, CA, USA) zu weißem Reis prozessiert. Für Untersuchungen der Kornzusammensetzung sowie der Stärkeeigenschaften wurden die weißen Körner mittels einer Labormühle (Cyclotec, Sample mill, Foss, Dänemark) zu sog. Reismehl vermahlen.
**5) Analyse der Expressionshöhe der Stärkesynthase II mittels Northern-Blot**
   Die Expression der Stärke-Synthase II aus Weizen in Reis wurde mittels Northern-Blot Analyse untersucht. Hierzu wurden für jedes unabhängige transgene Event drei unreife Reiskörner (ca. 15 Tage nach der Blüte) untersucht. Zur Homogenisierung wurden die gefrorenen Reiskörner in einer 96 Loch-Platte mit einer 4,5 mm Stahlkugel in einer Retsch-Mühle (Modell MM300) für 30 Sekunden bei einer Frequenz von 30 Hertz geschüttelt. Anschließend wurde die RNA mittels Promega RNA-Extraktionskit im 96-Loch-Maßstab nach Angaben des Herstellers isoliert (SV 96 Total RNA Isolation System, Bestell-Nr. Z3505, Promega, Mannheim).
   Je 2 µg RNA pro Probe wurden auf ein einheitliches Volumen gebracht und mit einem identischen Volumen RNA-Probenpuffer (65% (v/v) Formamid, 8% Formaldehyd, 13% (v/v) Gelpuffer (s. o.), 50 µg/ml Ethidiumbromid) versetzt. Nach Erhitzen (10 min, 65°C) und sofortigem Abkühlen auf Eis wurde die RNA über ein 1,2% (w/v) Agarosegel (20 mM MOPS pH 8,0, 5 mM Na-Acetat, 1 mM EDTA, 6% (v/v) Formaldehyd) unter Verwendung von RNA-Laufpuffer (20 mM MOPS pH 8,0, 5 mM Na-Acetat, 1 mM EDTA) bei einer konstanten Stromstärke von 50-80 mA für ca. 2 Stunden aufgetrennt.
   Nachfolgend wurde die RNA mittels Diffusionsblot unter Verwendung von 10x SSC (1,5 M NaCl, 150 mM Na-Citrat pH 7,0) auf Hybond-N-Membran transferiert und mittels UV-Bestrahlung auf der Membran immobilisiert.
   Für die Hybridisierung des Northern-Blots wurde ein ca. 1 kb Spel/BspHl-Fragment des Plasmids AH32-191 (Bp 4568-5686), welches den 5'-Bereich der SSII cDNA darstellt, verwendet. Die radioaktive Markierung des DNA-Fragmentes erfolgte mittels des Random primed DNA labeling Kit der Firma Roche (Bestell-Nr. 1004 760) unter Ver-. wendung von ³²P-α-dCTP nach Angaben des Herstellers.
   Der Northern-Blot wurde für vier Stunden bei 60°C unter leichtem Schütteln im Wasserbad mit Hybridisierungspuffer (250 mM Na-Phosphat-Puffer pH 7,2, 1 mM EDTA, 6% (w/v) SDS, 1 % (w/v) BSA) vorinkubiert, bevor zur Hybridisierung die radioaktiv markierte DNA hinzugefügt wurde. Nach 16 Stunden Inkubation wurde die Hybridisierungslösung entfernt und die Membran zur Entfernung unspezifisch gebundener DNA-Moleküle auf einander folgend mit 3xSSC und 2xSSC (s. o.) bei 60°C unter leichtem Schütteln im Wasserbad gewaschen.
   Zum Nachweis markierter RNA erfolgte eine Autoradiographie der Membran auf einen Röntgenfilm bei -70°C für ein bis drei Tage.
**6) Bestimmung der SSII-Aktivität mittels Aktivitätsgel**
   Der Nachweis der verschiedenen Stärke-Synthase-Aktivitäten in unreifen Reiskörnern erfolgte mittels Aktivitätsgelen (Zymogrammen), bei denen Proteinextrakte unter nativen Bedingungen in einem Polyacrylamidgel aufgetrennt und nachfolgend mit entsprechenden Substraten inkubiert werden. Das entstandene Reaktionsprodukt (Stärke) wurde mittels Lugol'scher Lösung im Gel angefärbt.
   Einzelne unreife Reiskörner (ca. 15 Tage nach Blüte) wurden in flüssigem Stickstoff schock gefroren und in 150-200 µl kaltem Extraktionspuffer (50 mM Tris/HCl pH 7,6, 2,5 mM EDTA, 2 mM DTT, 4 mM PMSF, 0,1% (w/v) Glykogen, 10% (v/v) Glyzerin) homogenisiert. Nach Zentrifugation (15 min, 13000 g, 4°C) wurde der klare Überstand in ein frisches Reaktionsgefäss überführt und ein Aliquot des Extraktes zur Bestimmung des Proteingehaltes nach Bradford (1976, Anal Biochem 72: 248-254) verwendet.
   Die Auftrennung der Proteinextrakte erfolgte mittels eines kontinuierlichen 7,5% Polyacrylamid-Geles (7,5% AA/BAA 37,5:1; 25 mM Tris/HCl pH 7,6, 192 mM Gylcin, 0,1% (w/v) APS, 0,05% (v/v) TEMED) unter Verwendung von einfach konzentriertem Laufpuffer (25 mM Tris/HCl, 192 mM Glycin). Für jede Probe wurden 15 µg Protein aufgetragen und die Elektrophorese für 2 - 2,5 Stunden bei 4°C durchgeführt.
   Nachfolgend wurden die Gele in 15 ml Inkubationspuffer (0,5 mM Natriumcitrat pH 7,0, 25 mM Kaliumacetat, 2 mM EDTA, 2 mM DTT, 0,1% (w/v) Amylopektin, 50 mM Tricine/NaOH pH 8,5, 1 mM ADP-Glucose) über Nacht bei Raumtemperatur unter ständigem Schütteln inkubiert. Die Anfärbung der gebildeten Stärke erfolgte mittels Lugol'scher Lösung.
**7) Bestimmung des Phosphatgehaltes in C6-Position (C6-P-Gehalt):**
   In der Stärke können die Positionen C3 und C6 der Glukoseeinheiten phosphoryliert sein. Zur Bestimmung des C6-P-Gehaltes der Stärke (modifiziert nach Nielsen et al., 1994, Plant Physiol. 105: 111-117) wurden 50 mg Reismehl in 500 µl 0,7 M HCl 4 h bei 95°C unter ständigem Schütteln hydrolysiert. Anschließend wurden die Ansätze für 10 min bei 15.500 g zentrifugiert und die Überstände mittels einer Filtermembran (0,45 µM) von Schwebstoffen und Trübungen gereinigt. Von dem klaren Hydrolysat wurden 20 µl mit 180 µl lmidazol-Puffer (300 mM Imidazol, pH 7,4; 7,5 mM M_{g}Cl₂, 1 mM EDTA und 0,4 mM NADP) gemischt. Die Messung wurde im Photometer bei 340 nm durchgeführt. Nach Erfassung der Basisabsorption wurde die Enzymreaktion durch die Zugabe von 2 Einheiten (units) Glukose-6-Phosphat Dehydrogenase (von *Leuconostoc mesenteroides,* Boehringer Mannheim) gestartet. Die Absorptionsänderung beruht auf einer äquimolaren Umsetzung von Glukose-6-Phosphat und NADP zu 6-Phosphoglukonat und NADPH, wobei die Bildung des NADPH bei der o. g. Wellenlänge erfasst. wird. Die Reaktion wurde bis zum Erreichen eines Plateaus verfolgt. Das Ergebnis dieser Messung liefert den Gehalt an Glukose-6-Phosphat pro Menge Frischgewicht.
   Aus dem identischen Hydrolysat wurde anhand des Gehaltes an freigesetzter Glukose der Grad der Hydrolyse bestimmt. Dieser wird verwendet, um den Gehalt an Glukose-6-Phosphat auf den Anteil hydrolysierter Stärke aus der Menge Frischgewicht zu beziehen. Hierzu wurden 10 µl Hydrolysat mit 10 µl 0,7 M NaOH neutralisiert und nach-' folgend 1:200 mit Wasser verdünnt. 4 µl dieser Verdünnung wurden mit 196 µl Messpuffer (100 mM Imidazol pH 6,9, 5 mM MgCl₂, 1 mM ATP, 0,4 mM NADP) versetzt und zur Bestimmung der Basisabsorption verwendet. Die Reaktion wurde durch Zugabe von 2 µl Enzym-Mix (Hexokinase 1:10; Glukose-6-Phosphat Dehydrogenase aus Hefe 1:10 in Messpuffer) und bei 340nm bis zum Plateau verfolgt. Das Messprinzip entspricht dem der ersten Reaktion.

### Beispiel 1:

### Klonierungsschritte für die Herstellung eines Transformationsvektors zur Expression einer Stärkesynthase II aus Weizen in Reis

Verwendet wurden der Reis-Transformationsvektor IR103-123 (beschrieben in WO 05/030941) und das Plasmid CF31-191 (beschrieben in WO 97/45545 unter der Bezeichnung pTaSS1). Der Reistransformationsvektor IR103-123 dient der endospermspezifischen Expression des Zielgenes mittels des Globulin-Promotors aus Reis. In einem ersten Schritt a) erfolgt die Öffnung des Vektors IR103-123 mit den Restriktionsenzymen *EcoRV* und *Xhol.* Das Plasmid CF31-191 enthält die cDNA einer Stärke-Synthase II (SSII) aus Weizen (*Triticum aestivum*). In einem zweiten Schritt b) wird die cDNA der SSII mit den Restriktionsenzymen *Ecl136II* und *Xho*l aus dem Plasmid CF31-191 ausgeschnitten und über ein Agarosegel vom restlichen Vektor abgetrennt. Die Ligation des in Schritt a) geöffneten Vektors IR103-123 mit dem in Schritt b) isolierten Fragment aus dem Plasmid CF 31-191 liefert den Vektor AH32-191.

### Beispiel 2:

### Herstellung transgener Reispflanzen, die eine erhöhte SSII-Aktivität aufweisen

Zur Erzeugung transgener Pflanzen mit erhöhter SSII-Aktivität wurde die T-DNA des Plasmids AH32-191 mit Hilfe von Agrobakterien, wie bei Ishida et al. (1996, Nature Biotechnology 14 (6): 745-750) beschrieben, in Reispflanzen transferiert.

### Beispiel 3:

### Eigenschaften von Reisstärke verschiedener transgener Linien mit unterschiedlich hoher SSII-Aktivität

Von den gemäß Beispiel 2 hergestellten Pflanzen wurden Reiskörner geerntet, die anschließend mittels der oben beschriebenen Methode ("4) Prozessierung von Reiskörnern") zu Reismehl verarbeitet wurden. Die Stärkekomponente des Reismehls wurde anschließend mittels der oben beschriebenen Methode ("7) Bestimmung des Phosphatgehaltes in C6-Position (C6-P-Gehalt)") auf ihren Phosphatgehalt in C6-Position untersucht:

**Tabelle1:**

| *Linie* | *Expression SS2 (xfach WT)* | *C-6-P (in % WT)* |
|---|---|---|
| Wildtyp | 1 | 100 |
| GAOS0353-01301 | 6 | 184 |
| GAOS0353-02501 | 10 | 358 |

Tabelle 1: Eigenschaften von Reisstärke mit erhöhter SSII-Aktivität im Vergleich zum Wildtyp (WT). Aufgeführt sind die SSII-Aktivität (als Vielfaches des Wildtyps) und die Gehalte an Stärke-gebundenem Glukose-6-Phosphat (C-6-P).

Es zeigte sich, dass die Höhe der Aktivität der Stärkesynthase II mit der Höhe des Phosphatgehaltes in C6-Position korreliert. Die Linie GAOS0353-01301 zeigt eine um den Faktor 6 erhöhte Expression, der G-6-P-Gehalt hat sich im Vergleich zum Wildtyp fast verdoppelt. Die stärkste Ausprägung zeigt die Linie GAOS0353-02501 mit einer um den Faktor 10 erhöhten Expression der SSII sowie einer Erhöhung des C-6-P-Gehaltes auf über 350% im Vergleich zum Wildtyp (100%).

## Patentansprüche

1. Verfahren zur Erhöhung des Phosphatgehaltes von Stärken genetisch modifizierter Pflanzenzellen auf 150 bis 500% im Vergleich zu Stärken aus entsprechenden genetisch nicht modifizierten Wildtyppflanzenzellen (100%),
**dadurch gekennzeichnet, dass**
a) eine Pflanzenzelle genetisch modifiziert wird durch die Einführung eines fremden Nukleinsäuremoleküls kodierend eine lösliche Stärkesynthase II und
b) diese lösliche Stärkesynthase II überexprimiert wird.

2. Verfahren nach Anspruch 1, **dadurch gekennzeichnet, dass** es sich bei dem fremden Nukleinsäuremolekül um die kodierende Region einer heterologen löslichen Stärkesynthase II handelt.

3. Verfahren nach Anspruch 1, **dadurch gekennzeichnet, dass** es sich bei der löslichen Stärkesynthase II um eine lösliche Stärkesynthase II aus einer Monokotylenpflanze handelt.

4. Verfahren nach Anspruch 1, **dadurch gekennzeichnet, dass** es sich bei der löslichen Stärkesynthase II um eine lösliche Stärkesynthase II aus Weizen handelt.

5. Verfahren nach Anspruch 1, **dadurch gekennzeichnet, dass** die angegebene lösliche Stärkesynthase II die unter Seq ID No. 1 angegebene Nukleotidsequenz aufweist.

6. Verfahren nach einem der Ansprüche 1 bis 5, **dadurch gekennzeichnet, dass** die genetisch modifizierter Pflanzenzellen Kartoffel-, Mais- oder Reispflanzenzellen sind.


SEQUENCE LISTING

<110> Bayer CropScience GmbH

<120> Überexpression von Stärkesynthase in Pflanzen

<130> BCS 05-5006-EP

<160> 5

<170> PatentIn version 3.1

<210> 1
<211> 3006
<212> DNA
<213> Triticum aestivum

<220>
<221> CDS
<222> (227)..(2623)
<223>

<220>
<221> Region1
<222> (1190)..(1279)
<223>

<220>
<221> Region2
<222> (1493)..(1612)
<223>

<220>
<221> Region3

<222>  (2147)..(2350)

<223>

<300>

<302>  Nucleic acid molecules encoding enzymes from wheat which are involved in
starch synthesis

<308>  Derwent/AAV01528

<309>  1998-05-21

<310>  wo 97 45545

<311>  1997-05-28

<312>  1997-12-04

<313>  (1)..(2825)

<400>  1
   ctccaccgcg gtggcggccg ctctagaact agtggatccc ccgggctgca ggaattcggc      60
   acgagcttcg gcctgacccc gttcgtttac ccccacacag agcacactcc agtccagtcc     120
   agcccactgc caccgcgcta ctctccactc ccactgccac cacctccgcc tgcgccgcgc     180
   tctgggcgga ccaacccgcg aaccgtacca tctcccgccc cgatcc atg tcg tcg        235
                                                      Met Ser Ser
                                                      1
   gcg gtc gcg tcc gcc gca tcc ttc ctc gcg ctc gcg tca gcc tcc ccc       283
   Ala Val Ala Ser Ala Ala Ser Phe Leu Ala Leu Ala Ser Ala Ser Pro
       5                   10                  15
   ggg aga tca cgc agg cgg gcg agg gtg agc gcg cag cca ccc cac gcc       331
   Gly Arg Ser Arg Arg Arg Ala Arg Val Ser Ala Gln Pro Pro His Ala
   20                  25                  30                  35
   ggg gcc ggc agg ttg cac tgg ccg ccg tgg ccg ccg cag cgc acg gct       379
   Gly Ala Gly Arg Leu His Trp Pro Pro Trp Pro Pro Gln Arg Thr Ala
                   40                  45                  50
   cgc gac gga gct gtg gcg gcg ctc gcc gcc ggg aag aag gac gcg ggg       427
   Arg Asp Gly Ala Val Ala Ala Leu Ala Ala Gly Lys Lys Asp Ala Gly
               55                  60                  65
   atc gac gac gcc gcc gcg tcc gtg agg cag ccc cgc gca ctc cgc ggt       475
   Ile Asp Asp Ala Ala Ala Ser Val Arg Gln Pro Arg Ala Leu Arg Gly
           70                  75                  80
   ggc gcc gcc acc aag gtc gcg gag cga agg gat ccc gtc aag acg ctc       523
   Gly Ala Ala Thr Lys Val Ala Glu Arg Arg Asp Pro Val Lys Thr Leu
       85                  90                  95
   gac cgc gac gcc gcg gaa ggc ggc ggg ccg tcc ccg ccg gca gcg agg       571
   Asp Arg Asp Ala Ala Glu Gly Gly Gly Pro Ser Pro Pro Ala Ala Arg
   100                 105                 110                 115
   cag gac gcc gcc cgt ccg ccg agt atg aac ggc atg ccg gtg aac ggc       619
   Gln Asp Ala Ala Arg Pro Pro Ser Met Asn Gly Met Pro Val Asn Gly
                   120                 125                 130
   gag aac aaa tct acc ggc ggc ggc ggc gcg act aaa gac agc ggg ctg      667
   Glu Asn Lys Ser Thr Gly Gly Gly Gly Ala Thr Lys Asp Ser Gly Leu
               135                 140                 145
   ccc acg ccc gca cgc gcg ccc cat ccg tcg acc cag aac aga gca ccg 715
   Pro Thr Pro Ala Arg Ala Pro His Pro Ser Thr Gln Asn Arg Ala Pro
   150 155 160
   gtg aac ggt gaa aac aaa gct aac gtc gcc tcg ccg ccg acg agc ata 763
   Val Asn Gly Glu Asn Lys Ala Asn Val Ala Ser Pro Pro Thr Ser Ile
   165 170 175
   gcc gag gcc gcg gct tcg gat tcc gca gct acc att tcc atc agc gac 811
   Ala Glu Ala Ala Ala Ser Asp Ser Ala Ala Thr Ile Ser Ile Ser Asp
   180 185 190 195
   aag gcg ccg gag tcc gtt gtc cca gct gag aag acg ccg ccg tcg tcc 859
   Lys Ala Pro Glu Ser Val Val Pro Ala Glu Lys Thr Pro Pro Ser Ser
   200 205 210
   ggc tca aat ttc gag tcc tcg gcc tct gct ccc ggg tct gac act gtc 907
   Gly Ser Asn Phe Glu Ser Ser Ala Ser Ala Pro Gly Ser Asp Thr Val
   215 220 225
   agc gac gtg gaa caa gaa ctg aag aag ggt gcg gtc gtt gtc gaa gaa 955
   Ser Asp Val Glu Gln Glu Leu Lys Lys Gly Ala Val Val Val Glu Glu
   230 235 240
   gct cca aag cca aag gct ctt tcg ccg cct gca gcc ccc gct gta caa 1003
   Ala Pro Lys Pro Lys Ala Leu Ser Pro Pro Ala Ala Pro Ala Val Gln
   245 250 255
   gaa gac ctt tgg gat ttc aag aaa tac att ggt ttc gag gag ccc gtg 1051
   Glu Asp Leu Trp Asp Phe Lys Lys Tyr Ile Gly Phe Glu Glu Pro Val
   260 265 270 275
   gag gcc aag gat gat ggc cgg gct gtc gca gat gat gcg ggc tcc ttt 1099
   Glu Ala Lys Asp Asp Gly Arg Ala Val Ala Asp Asp Ala Gly Ser Phe
   280 285 290
   gaa cac cac cag aat cac gac tcc gga cct ttg gca ggg gag aat gtc 1147
   Glu His His Gln Asn His Asp Ser Gly Pro Leu Ala Gly Glu Asn Val
   295 300 305
   atg aac gtg gtc gtc gtg gct gct gag tgt tct ccc tgg tgc aaa aca 1195
   Met Asn Val Val Val Val Ala Ala Glu Cys Ser Pro Trp Cys Lys Thr
   310 315 320
   ggt ggt ctg gga gat gtt gcg ggt gct ctg ccc aag gct ttg gca aag 1243
   Gly Gly Leu Gly Asp Val Ala Gly Ala Leu Pro Lys Ala Leu Ala Lys
   325 330 335
   aga gga cat cgt gtt atg gtt gtg gta cca agg tat ggg gac tat gaa 1291
   Arg Gly His Arg Val Met Val Val Val Pro Arg Tyr Gly Asp Tyr Glu
   340 345 350 355
   gaa gcc tac gat gtc gga gtc cga aaa tac tac aag gct gct gga cag 1339
   Glu Ala Tyr Asp Val Gly Val Arg Lys Tyr Tyr Lys Ala Ala Gly Gln
   360 365 370
   gat atg gaa gtg aat tat ttc cat gct tat atc gat gga gtt gat ttt 1387
   Asp Met Glu Val Asn Tyr Phe His Ala Tyr Ile Asp Gly Val Asp Phe
   375 380 385
   gtg ttc att gac gct cct ctc ttc cga cac cgt cag gaa gac att tat 1435
   Val Phe Ile Asp Ala Pro Leu Phe Arg His Arg Gln Glu Asp Ile Tyr
   390 395 400
   ggg ggc agc aga cag gaa att atg aag cgc atg att ttg ttc tgc aag 1483
   Gly Gly Ser Arg Gln Glu Ile Met Lys Arg Met Ile Leu Phe Cys Lys
   405 410 415
   gcc gct gtt gag gtt cca tgg cac gtt cca tgc ggc ggt gtc cct tat 1531
   Ala Ala Val Glu Val Pro Trp His Val Pro Cys Gly Gly Val Pro Tyr
   420 425 430 435
   ggg gat gga aat ctg gtg ttt att gca aat gat tgg cac acg gca ctc 1579
   Gly Asp Gly Asn Leu Val Phe Ile Ala Asn Asp Trp His Thr Ala Leu
   440 445 450
   ctg cct gtc tat ctg aaa gca tat tac agg gac cat ggt ttg atg cag 1627
   Leu Pro Val Tyr Leu Lys Ala Tyr Tyr Arg Asp His Gly Leu Met Gln
   455 460 465
   tac act cgg tcc att atg gtg ata cat aac atc gct cac cag ggc cgt 1675
   Tyr Thr Arg Ser Ile Met Val Ile His Asn Ile Ala His Gln Gly Arg
   470 475 480
   ggc cct gta gat gaa ttc ccg ttc acc gag ttg cct gag cac tac ctg 1723
   Gly Pro Val Asp Glu Phe Pro Phe Thr Glu Leu Pro Glu His Tyr Leu
   485 490 495
   gaa cac ttc aga ctg tac gac ccc gtg ggt ggt gaa cac gcc aac tac 1771
   Glu His Phe Arg Leu Tyr Asp Pro Val Gly Gly Glu His Ala Asn Tyr
   500 505 510 515
   ttc gcc gcc ggc ctg aag atg gcg gac cag gtt gtc gtg gtg agc ccc 1819
   Phe Ala Ala Gly Leu Lys Met Ala Asp Gln Val Val Val Val Ser Pro
   520 525 530
   ggg tac ctg tgg gag ctg aag acg gtg gag ggc ggc tgg ggg ctt cac 1867
   Gly Tyr Leu Trp Glu Leu Lys Thr Val Glu Gly Gly Trp Gly Leu His
   535 540 545
   gac atc ata cgg cag aac gac tgg aag acc cgc ggc atc gtc aac ggc 1915
   Asp Ile Ile Arg Gln Asn Asp Trp Lys Thr Arg Gly Ile Val Asn Gly
   550 555 560
   atc gac aac atg gag tgg aac ccc gag gtg gac gcc cac ctc aag tcg 1963
   Ile Asp Asn Met Glu Trp Asn Pro Glu Val Asp Ala His Leu Lys Ser
   565 570 575
   gac ggc tac acc aac ttc tcc ctg agg acg ctg gac tcc ggc aag cgg 2011
   Asp Gly Tyr Thr Asn Phe Ser Leu Arg Thr Leu Asp Ser Gly Lys Arg
   580 585 590 595
   cag tgc aag gag gcc ctg cag cgc gag ctg ggc ctg cag gtc cgc gcc 2059
   Gln Cys Lys Glu Ala Leu Gln Arg Glu Leu Gly Leu Gln Val Arg Ala
   600 605 610
   gac gtg ccg ctg ctc ggc ttc atc ggc cgc ctg gac ggg cag aag ggc 2107
   Asp Val Pro Leu Leu Gly Phe Ile Gly Arg Leu Asp Gly Gln Lys Gly
   615 620 625
   gtg gag atc atc gcg gac gcc atg ccc tgg atc gtg agc cag gac gtg 2155
   Val Glu Ile Ile Ala Asp Ala Met Pro Trp Ile Val Ser Gln Asp Val
   630 635 640
   cag ctg gtg atg ctg ggc acc ggg cgc cac gac ctg gag agc atg ctg 2203
   Gln Leu Val Met Leu Gly Thr Gly Arg His Asp Leu Glu Ser Met Leu
   645 650 655
   cag cac ttc gag cgg gag cac cac gac aag gtg cgc ggg tgg gtg ggg 2251
   Gln His Phe Glu Arg Glu His His Asp Lys Val Arg Gly Trp Val Gly
   660 665 670 675
   ttc tcc gtg cgc ctg gcg cac cgg atc acg gcg ggg gcg gac gcg ctc 2299
   Phe Ser Val Arg Leu Ala His Arg Ile Thr Ala Gly Ala Asp Ala Leu
   680 685 690
   ctc atg ccc tcc cgg ttc gag ccg tgc ggg ctg aac cag ctc tac gcc 2347
   Leu Met Pro Ser Arg Phe Glu Pro Cys Gly Leu Asn Gln Leu Tyr Ala
   695 700 705
   atg gcc tac ggc acc gtc ccc gtc gtg cac gcc gtc ggc ggc ctc agg 2395
   Met Ala Tyr Gly Thr Val Pro Val Val His Ala Val Gly Gly Leu Arg
   710 715 720
   gac acc gtg ccg ccg ttc gac ccc ttc aac cac tcc ggg ctc ggg tgg 2443
   Asp Thr Val Pro Pro Phe Asp Pro Phe Asn His Ser Gly Leu Gly Trp
   725 730 735
   acg ttc gac cgc gcc gag gcg cac aag ctg atc gag gcg ctc ggg cac 2491
   Thr Phe Asp Arg Ala Glu Ala His Lys Leu Ile Glu Ala Leu Gly His
   740 745 750 755
   tgc ctc cgc acc tac cga gac ttc aag gag agc tgg agg gcc ctc cag 2539
   Cys Leu Arg Thr Tyr Arg Asp Phe Lys Glu Ser Trp Arg Ala Leu Gln
   760 765 770
   gag cgc ggc atg tcg cag gac ttc agc tgg gag cac gcc gcc aag ctc 2587
   Glu Arg Gly Met Ser Gln Asp Phe Ser Trp Glu His Ala Ala Lys Leu
   775 780 785
   tac gag gac gtc ctc gtc aag gcc aag tac cag tgg tgaacgctag 2633
   Tyr Glu Asp Val Leu Val Lys Ala Lys Tyr Gln Trp
   790 795
   ctgctagccg ctccagcccc gcatgcgtgc atgacaggat ggaactgcat tgcgcacgca 2693
   ggaaagtgcc atggagcgcc ggcatccgcg aagtacagtg acatgaggtg tgtgtggttg 2753
   agacgctgat tccaatccgg cccgtagcag agtagagcgg aggtatatgg gaatcttaac 2813
   ttggtattgt aatttgttat gttgtgtgca ttattacaat gttgttactt attcttgtta 2873
   agtcggaggc caagggcgaa agctagctca catgtctgat ggatgcacgt gccatggttg 2933
   gtttggtagc gcagtgcaaa cggcaagaat gggaagtgaa ttcctccctg cttgaaaaaa 2993
   aaaaaaaaaa aaa 3006

<210> 2

<211> 799

<212> PRT

<213> Triticum aestivum

<400> 2

Met Ser Ser Ala Val Ala Ser Ala Ala Ser Phe Leu Ala Leu Ala Ser
1               5                   10                  15

Ala Ser Pro Gly Arg Ser Arg Arg Arg Ala Arg Val Ser Ala Gln Pro
            20                  25                  30

Pro His Ala Gly Ala Gly Arg Leu His Trp Pro Pro Trp Pro Pro Gln

35                  40                  45

Arg Thr Ala Arg Asp Gly Ala Val Ala Ala Leu Ala Ala Gly Lys Lys
    50                  55                  60

Asp Ala Gly Ile Asp Asp Ala Ala Ala Ser Val Arg Gln Pro Arg Ala
65                  70                  75                  80

Leu Arg Gly Gly Ala Ala Thr Lys Val Ala Glu Arg Arg Asp Pro Val
                85                  90                  95

Lys Thr Leu Asp Arg Asp Ala Ala Glu Gly Gly Gly Pro Ser Pro Pro
            100                 105                 110

Ala Ala Arg Gln Asp Ala Ala Arg Pro Pro Ser Met Asn Gly Met Pro
        115                 120                 125

Val Asn Gly Glu Asn Lys Ser Thr Gly Gly Gly Gly Ala Thr Lys Asp
    130                 135                 140

Ser Gly Leu Pro Thr Pro Ala Arg Ala Pro His Pro Ser Thr Gln Asn
145                 150                 155                 160

Arg Ala Pro Val Asn Gly Glu Asn Lys Ala Asn Val Ala Ser Pro Pro
                165                 170                 175

Thr Ser Ile Ala Glu Ala Ala Ala Ser Asp Ser Ala Ala Thr Ile Ser
            180                 185                 190

Ile Ser Asp Lys Ala Pro Glu Ser Val Val Pro Ala Glu Lys Thr Pro
        195                 200                 205

Pro Ser Ser Gly Ser Asn Phe Glu Ser Ser Ala Ser Ala Pro Gly Ser
    210                 215                 220

Asp Thr Val Ser Asp Val Glu Gln Glu Leu Lys Lys Gly Ala Val Val
225                 230                 235                 240

Val Glu Glu Ala Pro Lys Pro Lys Ala Leu Ser Pro Pro Ala Ala Pro
                245                 250                 255

Ala Val Gln Glu Asp Leu Trp Asp Phe Lys Lys Tyr Ile Gly Phe Glu
            260                 265                 270

Glu Pro Val Glu Ala Lys Asp Asp Gly Arg Ala Val Ala Asp Asp Ala
        275                 280                 285

Gly Ser Phe Glu His His Gln Asn His Asp Ser Gly Pro Leu Ala Gly
    290                 295                 300

Glu Asn Val Met Asn Val Val Val Val Ala Ala Glu Cys Ser Pro Trp

305                 310                 315                 320

Cys Lys Thr Gly Gly Leu Gly Asp Val Ala Gly Ala Leu Pro Lys Ala
                325                 330                 335

Leu Ala Lys Arg Gly His Arg Val Met Val Val Val Pro Arg Tyr Gly
            340                 345                 350

Asp Tyr Glu Glu Ala Tyr Asp Val Gly Val Arg Lys Tyr Tyr Lys Ala
        355                 360                 365

Ala Gly Gln Asp Met Glu Val Asn Tyr Phe His Ala Tyr Ile Asp Gly
    370                 375                 380

Val Asp Phe Val Phe Ile Asp Ala Pro Leu Phe Arg His Arg Gln Glu
385                 390                 395                 400

Asp Ile Tyr Gly Gly Ser Arg Gln Glu Ile Met Lys Arg Met Ile Leu
                405                 410                 415

Phe Cys Lys Ala Ala Val Glu Val Pro Trp His Val Pro Cys Gly Gly
            420                 425                 430

Val Pro Tyr Gly Asp Gly Asn Leu Val Phe Ile Ala Asn Asp Trp His
        435                 440                 445

Thr Ala Leu Leu Pro Val Tyr Leu Lys Ala Tyr Tyr Arg Asp His Gly
    450                 455                 460

Leu Met Gln Tyr Thr Arg Ser Ile Met Val Ile His Asn Ile Ala His
465                 470                 475                 480

Gln Gly Arg Gly Pro Val Asp Glu Phe Pro Phe Thr Glu Leu Pro Glu
                485                 490                 495

His Tyr Leu Glu His Phe Arg Leu Tyr Asp Pro Val Gly Gly Glu His
            500                 505                 510

Ala Asn Tyr Phe Ala Ala Gly Leu Lys Met Ala Asp Gln Val Val Val
        515                 520                 525

Val Ser Pro Gly Tyr Leu Trp Glu Leu Lys Thr Val Glu Gly Gly Trp
    530                 535                 540

Gly Leu His Asp Ile Ile Arg Gln Asn Asp Trp Lys Thr Arg Gly Ile
545                 550                 555                 560

Val Asn Gly Ile Asp Asn Met Glu Trp Asn Pro Glu Val Asp Ala His
                565                 570                 575

Leu Lys Ser Asp Gly Tyr Thr Asn Phe Ser Leu Arg Thr Leu Asp Ser

            580                 585                 590

Gly Lys Arg Gln Cys Lys Glu Ala Leu Gln Arg Glu Leu Gly Leu Gln
        595                 600                 605

Val Arg Ala Asp Val Pro Leu Leu Gly Phe Ile Gly Arg Leu Asp Gly
    610                 615                 620

Gln Lys Gly Val Glu Ile Ile Ala Asp Ala Met Pro Trp Ile Val Ser
625                 630                 635                 640

Gln Asp Val Gln Leu Val Met Leu Gly Thr Gly Arg His Asp Leu Glu
                645                 650                 655

Ser Met Leu Gln His Phe Glu Arg Glu His His Asp Lys Val Arg Gly
            660                 665                 670

Trp Val Gly Phe Ser Val Arg Leu Ala His Arg Ile Thr Ala Gly Ala
        675                 680                 685

Asp Ala Leu Leu Met Pro Ser Arg Phe Glu Pro Cys Gly Leu Asn Gln
    690                 695                 700

Leu Tyr Ala Met Ala Tyr Gly Thr Val Pro Val Val His Ala Val Gly
705                 710                 715                 720

Gly Leu Arg Asp Thr Val Pro Pro Phe Asp Pro Phe Asn His Ser Gly
                725                 730                 735

Leu Gly Trp Thr Phe Asp Arg Ala Glu Ala His Lys Leu Ile Glu Ala
            740                 745                 750

Leu Gly His Cys Leu Arg Thr Tyr Arg Asp Phe Lys Glu Ser Trp Arg
        755                 760                 765

Ala Leu Gln Glu Arg Gly Met Ser Gln Asp Phe Ser Trp Glu His Ala
    770                 775                 780

Ala Lys Leu Tyr Glu Asp Val Leu Val Lys Ala Lys Tyr Gln Trp
785                 790                 795

<210> 3

<211> 30

<212> PRT

<213> Triticum aestivum

<400> 3

Lys Thr Gly Gly Leu Gly Asp Val Ala Gly Ala Leu Pro Lys Ala Leu

1               5                   10                  15

Ala Lys Arg Gly His Arg Val Met Val Val Val Pro Arg Tyr
            20                  25                  30

<210>  4

<211>  40

<212>  PRT

<213>  Triticum aestivum


<400>  4

Glu Val Pro Trp His Val Pro Cys Gly Gly Val Pro Tyr Gly Asp Gly
1               5                   10                  15

Asn Leu Val Phe Ile Ala Asn Asp Trp His Thr Ala Leu Leu Pro Val
            20                  25                  30

Tyr Leu Lys Ala Tyr Tyr Arg Asp
        35                  40

<210>  5

<211>  68

<212>  PRT

<213>  Triticum aestivum


<400>  5

Gln Asp Val Gln Leu Val Met Leu Gly Thr Gly Arg His Asp Leu Glu
1               5                   10                  15

Ser Met Leu Gln His Phe Glu Arg Glu His His Asp Lys Val Arg Gly
            20                  25                  30

Trp Val Gly Phe Ser Val Arg Leu Ala His Arg Ile Thr Ala Gly Ala
        35                  40                  45

Asp Ala Leu Leu Met Pro Ser Arg Phe Glu Pro Cys Gly Leu Asn Gln
    50                  55                  60

Leu Tyr Ala Met
65